# EUROPÄISCHE PATENTANMELDUNG

(11) **EP 3 485 926 A1**
(43) Veröffentlichungstag der Anmeldung: **22.05.2019**
(21) Anmeldenummer: 17202085.1
(22) Anmeldetag: 16.11.2017
(51) Int. Cl.: A61M 1/12, A61M 1/10

(54) **EINLASSKANÜLE FÜR EINE FLUIDPUMPE**

(71) Anmelder: Berlin Heart GmbH, 12247 Berlin (DE)
(72) Erfinder: WISNIEWSKI, Adrian, 10963 Berlin (DE)
(74) Vertreter: Pfenning, Meinig & Partner mbB

(57) **Zusammenfassung**

Die Erfindung betrifft eine Einlasskanüle zum Zuführen eines Fluids aus einem menschlichen Gefäß in eine Fluidpumpe, wobei die Einlasskanüle als zum Führen des Fluids geeignete Hohlstruktur ausgebildet ist und eine Oberfläche der Einlasskanüle in einer Flussrichtung des Fluids aufeinanderfolgend eine Einlasszone und eine Einwachszone aufweist, wobei die Einwachszone und die Einlasszone durch eine in Umfangsrichtung der Einlasskanüle verlaufende Abrisskante voneinander getrennt sind, wobei eine erste Tangente an die Einlasszone an der Abrisskante einen Winkel zu einer Längsachse der Einlasskanüle von > 0° und < 180 ° aufweist, und wobei eine Oberflächenrauheit in der Einwachszone größer ist als eine Oberflächenrauheit in der Einlasszone.

## Beschreibung

Die vorliegende Erfindung betrifft eine Einlasskanüle zum Zuführen eines Fluids aus einem menschlichen Gefäß sowie ein Fluidpumpensystem mit einer Einlasskanüle und einer Fluidpumpe.

Im Stand der Technik sind Einlasskanülen für Herzpumpen bekannt, welche Blut aus einem Ventrikel in eine Blutpumpe führen. Die Einlasskanülen weisen im Allgemeinen entlang ihrer äußeren Oberfläche einen strukturierten Bereich mit erhöhter Oberflächenrauheit auf. Die Strukturierung soll ein Einwachsen der Einlasskanüle in die Ventrikelwand ermöglichen. Im Bereich der Einlassöffnung sind Einlasskanülen im Allgemeinen poliert, sodass dort die Oberflächenrauheit minimal ist. Dies soll wiederum einen ungebremsten, gleichmäßigen Fluss des Blutes in die Einlasskanüle ermöglichen. Die im Stand der Technik bekannten Einlasskanülen weisen dennoch ein Design auf, welches ein hohes Risiko einer Thrombenbildung durch Störung des Blutflusses am Einlass der Einlasskanüle birgt. Weiterhin besteht bei den bekannten Einlasskanülen das Risiko, dass Thromben aus dem Ventrikel in die Einlasskanüle und dann auch in die Blutpumpe gelangen und diese verstopfen.

Eine Aufgabe der vorliegenden Erfindung ist es daher, eine Einlasskanüle für eine Fluidpumpe bereitzustellen, welche einen ungestörten und gleichmäßigen Fluidfluss in die Fluidpumpe ermöglicht sowie das Risiko einer Thrombenbildung und Thrombeneinleitung in die Fluidpumpe minimiert. Weiterhin ist es die Aufgabe der vorliegenden Erfindung ein Fluidpumpensystem mit einer derartigen Einlasskanüle sowie einer Fluidpumpe bereitzustellen.

Die Aufgabe wird durch eine Einlasskanüle gemäß Anspruch 1 sowie ein Fluidpumpensystem gemäß Anspruch 15 gelöst. Vorteilhafte Weiterbildungen der erfindungsgemäßen Einlasskanüle sind in den abhängigen Ansprüchen aufgeführt.

Eine erfindungsgemäße Einlasskanüle zum Zuführen eines Fluids, insbesondere von Blut, aus einem menschlichen Gefäß in eine Fluidpumpe ist als eine zum Führen des Fluids geeignete Hohlstruktur ausgebildet. Eine Oberfläche der Einlasskanüle weist in einer Flussrichtung des Fluids aufeinanderfolgend eine Einlasszone und eine Einwachszone auf.

Unter der Oberfläche der Einlasskanüle ist die gesamte Oberfläche der als Hohlstruktur ausgebildeten Einlasskanüle, also sowohl die dem menschlichen Gefäß zugewandte Außenoberfläche als auch die einem Inneren der Einlasskanüle zugewandte Innenoberfläche gemeint. Unter einer Flussrichtung des Fluids ist außerdem eine Flussrichtung mindestens eines Fluidanteils aus dem menschlichen Gefäß in die Einlasskanüle gemeint, insbesondere auch aus einem Bereich des menschlichen Gefäßes, welcher sich um die Außenoberfläche der Einlasskanüle befindet.

Bei der erfindungsgemäßen Einlasskanüle sind die Einwachszone und die Einlasszone durch eine in Umfangsrichtung der Einlasskanüle verlaufende Abrisskante voneinander getrennt, wobei eine erste Tangente an die Einlasszone an der Abrisskante einen Winkel zu einer Längsachse (im Weiteren auch alternativ als Mittelachse bezeichnet) der Einlasskanüle von > 0° und < 180 ° aufweist.

Unter der Abrisskante wird eine klare, geometrische Kante entlang des Umfangs der Einlasskanüle verstanden, welche aus der ansonsten glatten (von einer Oberflächenrauheit abgesehen) Außenoberfläche der Einlasskanüle herausragt. Die Abrisskante trennt Einwachszone und Einlasszone voneinander und gehört selbst weder zur Einwachs- noch zur Einlasszone. Diese klare, geometrische Kante sowie ein an die Abrisskante unmittelbar angrenzender Bereiche der Einlasszone kann von Oberflächenunebenheiten aufgrund einer materialspezifischen und/oder fertigungsbedingten Oberflächenrauheit überlagert sein. Die gemittelte Rautiefe dieser Oberflächenunebenheiten ist jedoch klein gegenüber einer gemittelten Höhe der Abrisskante, welche bspw. in Bezug auf ein Intervall um die Abrisskante bestimmt werden kann, deren Länge einer durchschnittlichen Dicke einer Wandung der Einlasskanüle entspricht. Die Abrisskante fertigungsbedingt einen maximalen Radius von bis zu 0,5 mm aufweisen.

Unter einer ersten Tangente an die Einlasszone an der Abrisskante wird eine Tangente in einem Bereich der Einlasszone verstanden, welcher unmittelbar an die Abrisskante angrenzt. Ferner wird unter einer ersten Tangente an die Abrisskante lediglich eine Tangente an die klare, geometrische Abrisskante verstanden und nicht an eine Kante aufgrund einer Oberflächenunebenheit.

Weiterhin ist bei der erfindungsgemäßen Einlasskanüle eine Oberflächenrauheit in der Einwachszone größer als eine Oberflächenrauheit in der Einlasszone.

Die Abrisskante der erfindungsgemäßen Einlasskanüle stellt somit eine klare, geometrische und nicht nur durch die Oberflächenrauheit definierte Trennung zwischen der Einwachszone und der Einlasszone dar. Durch die Abrisskante wird die Wandschubspannung in der Einlasszone im Bereich einer Einlassöffnung der Einlasskanüle erhöht, sodass ein geringeres Risiko der Thrombenbildung an der Einlassöffnung und der Einleitung von Thromben in die Einlasskanüle besteht.

insbesondere kann die erste Tangente einen Winkel zur Längsachse der Einlasskanüle zwischen 10° und 170°, vorzugsweise zwischen 30° und 160°, besonders bevorzugt zwischen 85° und 95°, insbesondere von 90°, oder zwischen 60° und 70° oder zwischen 110° und 130° oder zwischen 145° und 155° aufweisen. Die Einlasskanüle kann rotationssymmetrisch ausgebildet sein. In diesem Fall entspricht die Längsachse der Symmetrieachse der Rotationssymmetrie.

In einer vorteilhaften Ausgestaltung der erfindungsgemäßen Einlasskanüle kann die Einlasszone zumindest entlang eines Abstands von bis zu einer Hälfte der durchschnittlichen Dicke einer Wandung der Einlasskanüle von der Abrisskante in einem Winkel von > 0° und < 180 °, insbesondere zwischen 10° und 170°, vorzugsweise zwischen 30° und 160°, besonders bevorzugt zwischen 85° und 95°, insbesondere von 90°, oder zwischen 60° und 70° oder zwischen 110° und 130° oder zwischen 145° und 155° verlaufen.

In einer weiteren Ausgestaltung der erfindungsgemäßen Einlasskanüle verläuft die Abrisskante durchgängig in Umfangsrichtung der Einlasskanüle. Dies hat den Vorteil, dass um die gesamte Einlasskanüle herum ein möglichst ungestörter Fluidfluss und eine hohe Wandschubspannung zwischen dem Fluid und der Einlasszone gewährleistet ist.

Die erfindungsgemäße Einlasskanüle weist vorzugsweise ein hämokompatibles Material, insbesondere Titan, eine Titan-Legierung, insbesondere Ti₆Al₄V, Edelstahl und/oder Kunststoff auf oder besteht daraus.

In einer weiteren vorteilhaften Ausgestaltung der Erfindung weist die Einwachszone eine Texturierung auf. Dabei kann die Texturing lediglich in einem Teilbereich der Einwachszone vorhanden sein oder die Einwachszone vollständig ausfüllen. Die Texturierung kann mittels eines Sinterverfahrens, eines 3D-Druckverfahrens, insbesondere zum Aufdrucken einer Titan-Texturierung, durch Aufschießen von Kügelchen, insbesondere von Titan-Kügelchen, und/oder durch Aufkleben eines Fließstoffes, insbesondere Velours, erfolgen. Die Texturierung kann eine gemittelte Rautiefe von Rz > 16 µm aufweisen.

Weiterhin ist es vorteilhaft, wenn die Einlasszone poliert ist, wobei die polierte Einlasszone insbesondere eine gemittelte Rautiefe von Rz < 1,5 µm aufweisen kann.

Die Einwachszone kann ferner konkav, konvex oder nicht gekrümmt sein, während die Einlasszone konvex oder nicht gekrümmt sein kann. Dabei kann die Abrisskante einen Krümmungsübergang zwischen der Einwachszone und der Einlasszone bilden. Besonders bevorzugt sind dabei die folgenden Kombinationen der Krümmungen der Einwachszone und der Einlasszone: Die Einwachszone ist nicht gekrümmt, wobei sich die Einwachszone parallel zur Längsachse der Einlasskanüle erstreckt, und die Einlasszone ist konvex. Die Einwachszone ist konkav, und die Einlasszone ist konvex. Die Einwachszone und die Einlasszone sind konvex. Die Einwachszone ist konvex, und die Einlasszone ist nicht gekrümmt, wobei sich die Einlasszone in einem sich zur Einlassöffnung hin öffnenden Winkel zur Längsachse der Einlasskanüle erstreckt.

Die Einwachszone kann dabei mit einem Krümmungsradius von 2 bis 20 mm, bevorzugt 5,10 oder 15 mm konkav oder konvex gekrümmt sein.

Die Einlasszone kann mit einem Krümmungsradius von 2 bis 20 mm, bevorzugt 5,10 oder 15 mm konvex gekrümmt sein.

Des Weiteren ist es vorteilhaft, wenn sich die erste Tangente an die Einlasszone und eine zweite Tangente an die Einwachszone an der Abrisskante, in einem Winkel zwischen 40° und 150°, insbesondere in einem Winkel von 90° schneiden.

In einer vorteilhaften Ausführungsform der vorliegenden Erfindung kann bei einem mittleren Volumenstrom des Fluids von 2 bis 10 l/min, insbesondere 4,5 l/min, zwischen dem Fluid und der Einlasszone eine Wandschubspannung von 1 bis 10 Pa, insbesondere von 2 Pa, vorzugsweise von 4 Pa, vorliegen, und/oder in der Einwachszone eine Wandschubspannung von < 1 Pa vorliegen. Besonders vorteilhaft ist es, wenn sich beim Übergang von der Einwachszone zur Einlasszone, vorzugsweise in einem Bereich mit einem Abstand von der Abrisskante von bis zu 1 mm, ein Sprung in einer Wandschubspannung zwischen dem Fluid und der Oberfläche der Einlasskanüle von mindestens 100 % ergibt. Unmittelbar um die Abrisskante herum kann sich eine Fluidströmung auch etwas von der Oberfläche der Einlasskanüle ablösen, wodurch sich direkt an der Kante eine Absenkung der Wandschubspannung ergibt.

Insbesondere kann bei einem mittleren Volumenstrom des Fluids von 4,5 l/min in der Einwachszone in Bezug auf eine Flussrichtung des Fluids in einem Abstand von der Abrisskante von max. 0,75 mm eine Wandschubspannung von > 1 Pa vorliegen, in einem Abstand von der Abrisskante von max. 0,8 mm eine Wandschubspannung von < 0,25 Pa, in einem Abstand von der Abrisskante von max. 0,4 mm eine Wandschubspannung von < 0,5 Pa und in einem Abstand von der Abrisskante von max. 0,2 mm eine Wandschubspannung von < 0,75 Pa vorliegen. In der Einlasszone kann in Bezug auf eine Flussrichtung des Fluids unmittelbar hinter der Abrisskante eine Wandschubspannung von > 0,75 Pa, in einem Abstand von der Abrisskante von max. 0,75 mm eine Wandschubspannung von > 1 Pa, in einem Abstand von der Abrisskante von max. 1,5 mm eine Wandschubspannung von > 2Pa und in einem Abstand von der Abrisskante von max. 2,5 mm eine Wandschubspannung von > 4 Pa vorliegen.

Des Weiteren kann die Oberflächenrauheit in der Einwachszone in einem Abstand ≤ 5mm von der Abrisskante gleich der Oberflächenrauheit in der Einlasszone sein. Dies ist beispielsweise dann der Fall, wenn die Texturierung prozesstechnisch bedingt nicht bis direkt an die Abrisskante heran in der Einwachszone aufgebracht werden kann.

Zur Verbindung der Einlasskanülen mit einer Fluidpumpe kann die Einlasskanüle weiterhin eine Pumpenschnittstelle aufweisen, wobei die Pumpenschnittstelle außerhalb des menschlichen Gefäßes positionierbar ist, und an die Pumpenschnittstelle ein Pumpeneinlass der Fluidpumpe anschließbar ist.

Ferner ist es vorteilhaft, wenn eine dritte Tangente senkrecht zur Abrisskante an die Einlasszone in einem der Pumpenschnittstelle benachbarten Bereich der Einlasszone einen Winkel zwischen 0° und 30°, insbesondere von 10°, 15° oder 20° zur Längsachse der Einlasskanüle aufweist.

In einer weiteren vorteilhaften Ausgestaltung der Erfindung ist die Einlassöffnung der Einlasskanüle in einem der Abrisskante benachbarten Bereich der Einlasszone angeordnet, wobei in der Einlassöffnung ein Gitterelement angeordnet ist, welches die Einlassöffnung teilweise verschließt. Das Gitterelement kann einen Mittelteil und zwei, drei oder vier mit dem Mittelteil verbundene Arme aufweisen, wobei sich der Mittelteil entlang der Längsachse in der Mitte der Einlasskanüle erstreckt, und sich die Arme entlang der Einlasszone in die Einlasskanüle erstrecken. Das Gitterelement kann dazu dienen, einen Ansaugeffekt der Herzwand an der Einlasskanüle zu verhindern oder minimieren. Weiterhin wäre es denkbar, dass das Gitterelement verfestigte Fluidpartikel (z.B. Thromben für den Fall, dass es sich bei dem Fluid um Blut handelt, Trabekel, Gerinsel, Einwachsstrukturen oder Muskelgewebe) am Eindringen in die Einlasskanüle hindern kann.

Die Einlasskanüle kann insbesondere mit einer Fluidpumpe zusammenwirken, die ein nicht rotierendes, einlassseitiges, radial zentral angeordnetes Lagerelement aufweist. Das einlassseitige Lager kann radiale und axiale Kräfte und Momente aufnehmen. Das Gitterelement kann derart ausgebildet sein, dass der Mittelteil eine Nabe bildet, die sich entlang der Längsachse durch die Einlasskanüle erstreckt und einen feststehenden Teil Lagers der Fluidpumpe hält.

Die Erfindung umfasst weiterhin ein Fluidpumpensystem mit einer vorbeschriebenen Einlasskanüle und einer Fluidpumpe, wobei die Pumpenschnittstelle der Einlasskanüle an den Pumpeneinlass der Fluidpumpe angeschlossen ist.

Im Folgenden werden eine erfindungsgemäße Einlasskanüle und ein erfindungsgemäßes Fluidpumpensystem anhand von Figuren detaillierter beschrieben. Dabei werden verschiedene erfindungswesentliche oder auch vorteilhafte weiterbildende Elemente im Rahmen jeweils eines konkreten Beispiels genannt, wobei auch einzelne dieser Elemente als solche zur Weiterbildung der Erfindung - auch herausgelöst aus dem Kontext des jeweiligen Beispiels und weiterer Merkmale des jeweiligen Beispiels - verwendet werden können. Weiterhin werden in den Figuren für gleiche oder ähnliche Elemente gleiche oder ähnliche Bezugszeichen verwendet, und deren Erläuterung daher teilweise weggelassen.

Es zeigen
- Figur 1: eine schematische Darstellung eines Einwachsverhaltens und Blutflusses bei einer Einlasskanüle aus dem Stand der Technik,
- Figur 2: eine schematische Darstellung eines Einwachsverhaltens und Blutflusses bei einer erfindungsgemäßen Einlasskanüle,
- Figuren 3 bis 6: schematische Schnittansichten erfindungsgemäßer Einlasskanülen mit verschiedenen Geometrien und
- Figur 7: eine schematische Darstellung eines erfindungsgemäßen Fluidpumpensystems.

In den Figuren werden die folgenden Bezugszeichen verwendet:
- 1, 100: Einlasskanülen
- 11, 101: innerer Hohlraum
- 2, 120: Einwachszone
- 21, 121: Texturierung
- 3, 130: Einlasszone
- 4: Abrisskante
- 5, 110: Längsachse
- 6: erste Tangente
- 10: Fluidpumpe
- 12: Einlassöffnung
- 13: Pumpengehäuse
- 14: Gitterelement
- 14a: Mittelteil
- 14b: Arme
- 15: Fluidauslass
- 140: Ventrikelwand
- 141: Ventrikel
- 150: anwachsendes Gewebe
- 160: Thromben/Gewebe/Partikel
- 170: Blutfluss

Die im Folgenden beschriebenen Ausführungsbeispiele beziehen sich in erster Linie auf Einlasskanülen zum Zuführen von Blut aus einem Ventrikel in eine Blutpumpe.

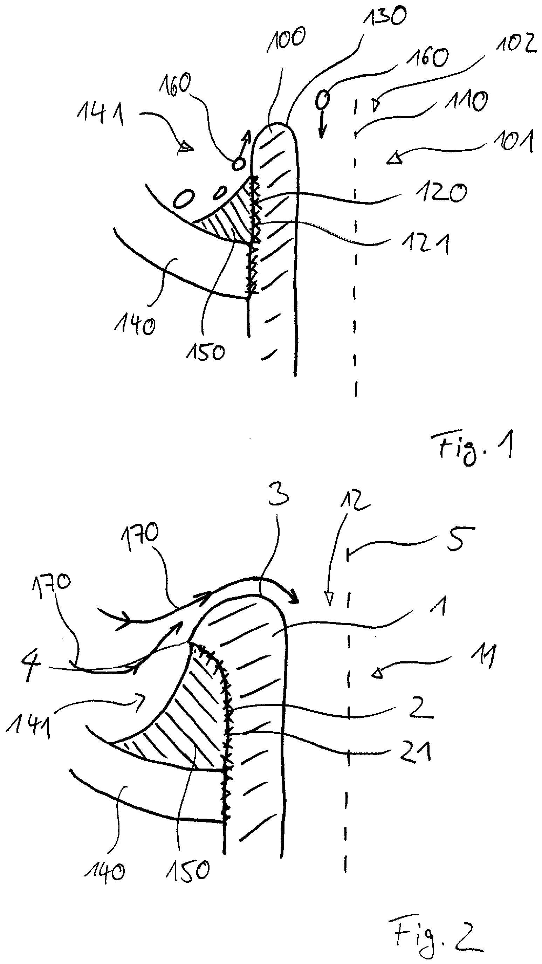

Figur 1 zeigt eine Einlasskanüle 100 gemäß dem Stand der Technik, die durch eine Öffnung in einer Ventrikelwand 140 in einen Ventrikel 141 eingeführt ist, in einer Schnittansicht parallel zu einer Längsachse 110 der Einlasskanüle 100. Die Einlasskanüle 100 ist hohlzylinderförmig ausgebildet. In Figur 1 ist lediglich ein Teil der Einlasskanüle 100 dargestellt. Die Einlasskanüle 100 weist einen inneren Hohlraum 101 auf und ist rotationssymmetrisch zur Längsachse 110. Auf einer zur Ventrikelwand 140 und zum Ventrikel 141 hin gelegenen Außenoberfläche weist die Einlasskanüle 100 eine Einwachszone 120 auf, welche mit einer Texturierung 121 versehen ist. Entlang der Einwachszone 120 ist ein Einwachsen der Einlasskanüle 100 erwünscht, weshalb in diesem Bereich die Texturierung 121 zur Erhöhung der Oberflächenrauheit aufgebracht ist. Im Bereich einer Einlassöffnung 102 der Einlasskanüle 100 erstreckt sich eine Einlasszone 130 von der Außenoberfläche über die Einlassöffnung 102 zu einer dem inneren Hohlraum 101 zugewandten Innenoberfläche. Im Unterschied zur Einwachszone 120 ist die Einlasszone 130 nicht texturiert. Die Einlasszone 130 ist vielmehr poliert, um die Oberflächenrauheit zu minimieren, um einen ungehinderten Blutfluss in diesem Bereich zu erhalten. Auf der Außenoberfläche gehen die Einwachszone 120 und die Einlasszone 130 fließend in Richtung des Flusses eines Teils des Blutes ineinander über. Eine Grenze zwischen der Einwachszone 120 und der Einlasszone 130 lässt sich lediglich aufgrund der wechselnden Oberflächenbeschaffenheit (texturiert/poliert) erkennen.

In Figur 1 ist ersichtlich, dass sich entlang der texturierten Einwachszone 120 Gewebe 150 gebildet hat, welches die Einlasskanüle 100 mit der Ventrikelwand 141 verbindet, sodass die Einlasskanüle 100 nicht mehr aus dem Ventrikel 140 herausrutschen kann. Im Bereich des angewachsenen Gewebes 150 ist jedoch der Blutfluss in Richtung der Einlasszone 130 der Einlasskanüle 100 gestört, was ein hohes Risiko der Bildung von Thromben 160 mit sich bringt. Diese Thromben 160 können dann zur Einlasszone 130 und ins Innere 101 der Einlasskanüle 100 gelangen. Von dort können diese Thromben 160 in eine angeschlossene Blutpumpe (in Figur 1 nicht gezeigt) gelangen und diese verstopfen oder blockieren. Des Weiteren kann auch anwachsendes Gewebe 150, welches über die Einwachszone hinaus wächst, von der Einwachszone 2 weiter in die Einlasszone 3 und von dort ins Innere der Einlasskanüle 100 gelangen und die angeschlossene Blutpumpe verstopfen oder blockieren.

Figur 2 zeigt eine ähnliche Situation wie in Figur 1 mit einer erfindungsgemäßen Einlasskanüle 1. Die erfindungsgemäße Einlasskanüle 1 ist hohlzylinderförmig ausgebildet und weist eine Einlassöffnung 12 sowie einen inneren Hohlraum 11 auf. Im Gegensatz zu der im Stand der Technik bekannten Einlasskanüle 100 ist die Einlassöffnung 12 der Einlasskanüle 11 an ihrer Außenoberfläche trompetenförmig ausgestellt. Die zum Inneren des Ventrikels 141 und zur Ventrikelwand 140 hin gelegene Außenoberfläche der Einlasskanüle 1 weist in Flussrichtung zumindest eines Teils des Blutes ebenfalls eine Einwachszone 2 und eine Einlasszone 3 auf, wobei sich die Einlasszone 3 von der Außenoberfläche der Einlasskanüle 1 über die Einlassöffnung 12 zur zum inneren Hohlraum 11 hin gelegenen Innenoberfläche erstreckt. Im Gegensatz zur Einlasskanüle 100 des Standes der Technik sind die Einwachszone 2 und die Einlasszone 3 durch eine sich in Umfangsrichtung der Einlasskanüle 1 erstreckende Abrisskante 4 klar voneinander getrennt. Die Einwachszone 2 weist zur Förderung des Einwachsens eine Texturierung 21 auf und ist konkav gekrümmt. Die Einlasszone 3 ist zur Verbesserung des Blutflusses poliert und weist eine konvexe Krümmung auf. Die Abrisskante 4 stellt somit einen unvermittelten Krümmungsübergang zwischen konkaver Krümmung der Einwachszone 2 und konvexer Krümmung der Einlasszone 3 dar.

In Figur 2 ist ersichtlich, dass sich ähnlich wie in Figur 1 Gewebe 150 entlang der Einwachszone 2 gebildet hat, wodurch die Einlasskanüle 1 nicht mehr aus dem Ventrikel 141 herausrutschen kann. Das Gewebe 150 ist bis zur Abrisskante 4 sauber angewachsen und bildet somit eine strömungstechnisch vorteilhafte Verbindung zwischen der zum Inneren des Ventrikels 141 zugewandten Innenoberfläche der Ventrikelwand 140 und der Einlasszone 3. Dies führt zu einer deutlich höheren Wandschubspannung zwischen dem Blut und der Einlasszone 3 im Bereich der Einlassöffnung 12 und zu einem ungestörten Blutfluss 170 aus einem der Einwachszone 2 benachbarten Randbereich des Ventrikels 141 in die Einlasskanüle 1. Somit wird bei der erfindungsgemäßen Einlasskanüle 1 durch die Abrisskante 4 ein Einwachsen der Einlasskanüle über die Einwachszone 3 hinaus und damit ein Risiko, dass Gewebe 150 in die Einlasskanüle 1 gelangt, verhindert. Thromben und andere Partikel (Trabekel, Gewebepartikel) werden außerdem durch die Abrisskante 4 in dem Randbereich des Ventrikels 141 gehalten und gelangen nicht in die Einlasskanüle 1. Auch das Risiko der Bildung von Thromben kann gegenüber der Einlasskanüle 100 des Standes der Technik verringert werden.

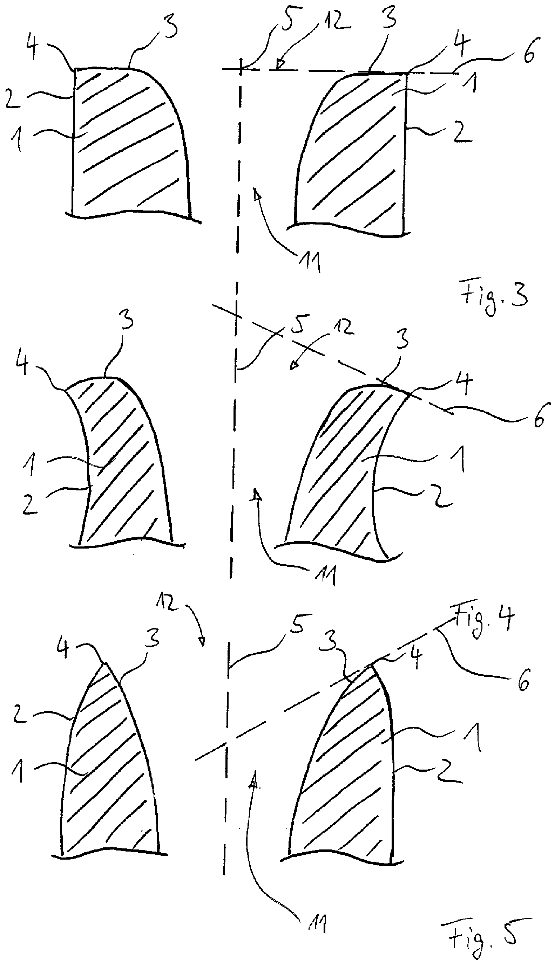

Figur 3 zeigt ein weiteres Ausführungsbeispiel einer erfindungsgemäßen Einlasskanüle 1 in einer schematischen Schnittansicht parallel zur Längsachse 5. Die Einlasskanüle 1 ist als offene Hohlstruktur mit einer zylinderförmigen Außenoberfläche ausgebildet. Entlang der Außenoberfläche weist die Einlasskanüle 1 eine nicht gekrümmte Einwachszone 2 auf. Im Bereich der Einlassöffnung 12 wird die Einwachszone 2 durch eine in Umfangsrichtung verlaufende Abrisskante 4 begrenzt. Von der Abrisskante 4 zu einer einem inneren Hohlraum 11 der Einlasskanüle 1 zugewandten Innenoberfläche erstreckt sich eine Einlasszone 3. Die Einlasszone 3 ist konvex gekrümmt, sodass sich ein Innendurchmesser der Einlasskanüle von der Einlassöffnung 12 wegführend verringert. Die Abrisskante 4 ist ferner derart ausgebildet, dass eine Tangente 6 an die Einlasszone 3 an der Abrisskante 4 in einem Winkel von etwa 90° zur Längsachse 5 verläuft.

Figur 4 zeigt ein weiteres Ausführungsbeispiel einer erfindungsgemäßen Einlasskanüle 1 in einer ähnlichen Darstellung wie in Figur 3. Im Gegensatz zur Einlasskanüle 1 der Figur 3 weist die Einwachszone 2 der Einlasskanüle 1 in Figur 4 eine konkave Krümmung auf, während die Einlasszone 3 ebenfalls konvex gekrümmt ist. Die Abrisskante 4 stellt somit einen unvermittelten Krümmungsübergang zwischen der konkaven Krümmung in der Einwachszone 2 und der konvexen Krümmung in der Einlasszone 3 dar. Die Abrisskante 4 ist ferner derart ausgebildet, dass eine Tangente 6 an die Einlasszone an der Abrisskante in einem zum inneren Hohlraum 11 hin offenen Winkel von etwa 65 ° zur Längsachse 5 verläuft. Ferner verjüngt sich der Innendurchmesser der Einlasskanüle 1 je größer der Abstand zur Einlassöffnung 12 ist.

Figur 5 zeigt ein weiteres Ausführungsbeispiel einer erfindungsgemäßen Einlasskanüle 1 in einer ähnlichen Darstellung wie in Figuren 3 und 4. Im Gegensatz zur Einlasskanüle 1 der Figuren 3 und 4 weisen die Einwachszone 2 und die Einlasszone 3 der Einlasskanüle 1 in Figur 5 eine konvexe Krümmung auf. Die Abrisskante 4 stellt somit einen unvermittelten Krümmungsübergang zwischen den beiden konvexen Krümmungen dar. Außerdem ist die Abrisskante 4 zur Mittelachse 5 der Einlasskanüle 1 hin verschoben, sodass ein Durchmesser der Einlasskanüle 1 auf Höhe der Abrisskante 4 zwischen einem maximalen Außendurchmesser und einem Innendurchmesser der Einlasskanüle 1 liegt. Die Abrisskante 4 ist weiterhin derart ausgebildet, dass eine Tangente 6 an die Einlasszone 3 an der Abrisskante 4 in einem zum inneren Hohlraum 11 hin offenen Winkel von etwa 120° zur Längsachse 5 verläuft. Ferner verjüngt sich der Innendurchmesser der Einlasskanüle 1 je größer der Abstand zur Einlassöffnung 12 ist.

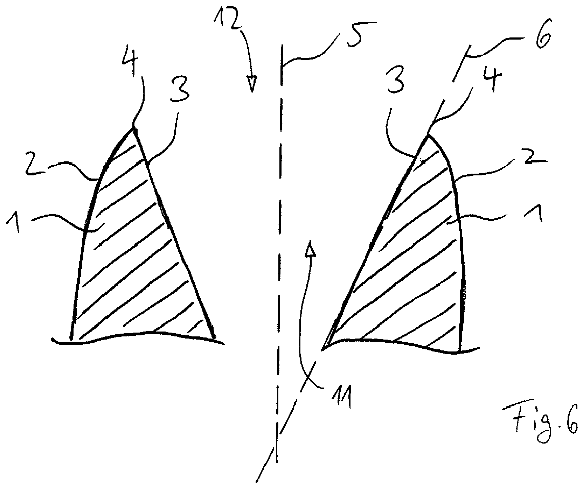

Figur 6 zeigt ein weiteres Ausführungsbeispiel einer erfindungsgemäßen Einlasskanüle 1 in einer ähnlichen Darstellung wie in den Figuren 3 bis 5. Die Einwachszone 2 der Einlasskanüle 1 weist in Figur 5 eine konvexe Krümmung auf, während die Einlasszone 3 nicht gekrümmt ist. Die Abrisskante 4 stellt somit einen Krümmungsübergang zwischen der konvexen Krümmung in der Einwachszone 2 und der nicht vorhandenen Krümmung in der Einlasszone 3 dar. Die Abrisskante 4 ist weiterhin derart ausgebildet, dass eine Tangente 6 an die Einlasszone 3 an der Abrisskante 4 in einem sich zum inneren Hohlraum 11 hin offenen Winkel von etwa 155° zur Längsachse 5 verläuft. Die Einlasszone 3 verläuft in einem sich zur Einlassöffnung 12 hin öffnenden, spitzen Winkel zur Längsachse 5. Dadurch verjüngt sich der Innendurchmesser der Einlasskanüle 1 je größer der Abstand zur Einlassöffnung 12 ist. Ähnlich wie in Figur 5 ist die Abrisskante 4 der Einlasskanüle 1 in Figur 6 zur Mittelachse 5 der Einlasskanüle 1 hin verschoben, sodass ein Durchmesser der Einlasskanüle 1 auf Höhe der Abrisskante 4 zwischen einem maximalen Außendurchmesser und einem Innendurchmesser der Einlasskanüle 1 liegt.

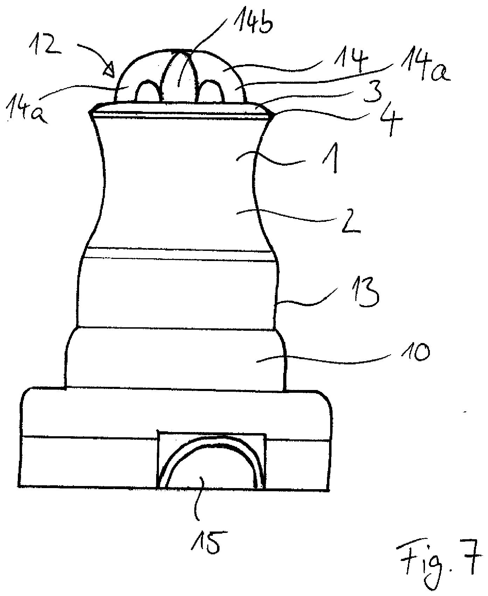

Figur 7 zeigt eine schematische Darstellung eines erfindungsgemäßen Fluidpumpensystems umfassend eine Einlasskanüle 1 und eine Fluidpumpe 10. Die Einlasskanüle 1 weist eine Einwachszone 2 und eine Einlasszone 3 auf, welche im Bereich der Einlassöffnung 12 durch eine Abrisskante 4 voneinander getrennt sind. In der Einlassöffnung 12 ist ein Gitterelement 14 angeordnet, welches aus der Einlasskanüle 1 herausragt und die Einlassöffnung 12 teilweise verschließt. Das Gitterelement 14 weist zwei gegenüberliegende Arme 14a sowie einen Mittelteil 14b auf, welcher die Arme 14a miteinander verbindet. Die Arme 14a erstrecken sich jeweils bogenförmig vom Mittelteil 14b zur Innenoberfläche der Einlasskanüle 1. Der Mittelteil 14b bildet ferner eine Nabe (hier nicht gezeigt), die sich entlang der Mittelachse 5 (Längsachse) der Einlasskanüle 1 in den inneren Hohlraum 11 der Einlasskanüle 1 erstreckt. Der als Nabe ausgebildete Mittelteil 14b hält im inneren Hohlraum 11 einen feststehenden Teil des Lagers der Fluidpumpe 10. Die Arme 14a erstrecken sich in Richtung der Längsachse 5 entlang der Innenoberfläche der Einlasskanüle 1 und stützen sich an der Innenoberfläche ab. Das Gitterelement 14 dient ferner dazu, einen Ansaugeffekt einer Herzwand an die Einlassöffnung zu verhindern oder zu minimieren. Weiterhin ist es denkbar, dass durch das Gitterelement zusätzlich ein Einströmen von sich möglicherweise im Bereich der Einlasskanüle befindlichen Thromben gehemmt werden kann. Auf einer der Einlassöffnung 12 abgewandten Seite der Einlasskanüle 1 ist ein Pumpengehäuse 13 der Fluidpumpe 10 angeschlossen. Bei der Fluidpumpe 10 handelt es sich insbesondere um eine Pumpe, welche das Fluid in Richtung der Längsachse 5 von der Einlassöffnung 12 zu einem Fluidauslass 15 fördert.

## Patentansprüche

1. Einlasskanüle zum Zuführen eines Fluids aus einem menschlichen Gefäß in eine Fluidpumpe, wobei die Einlasskanüle als zum Führen des Fluids geeignete Hohlstruktur ausgebildet ist und
eine Oberfläche der Einlasskanüle in einer Flussrichtung des Fluids aufeinanderfolgend eine Einlasszone und eine Einwachszone aufweist,
wobei die Einwachszone und die Einlasszone durch eine in Umfangsrichtung der Einlasskanüle verlaufende Abrisskante voneinander getrennt sind, wobei eine erste Tangente an die Einlasszone an der Abrisskante einen Winkel zu einer Längsachse der Einlasskanüle von > 0° und < 180 ° aufweist, und
wobei eine Oberflächenrauheit in der Einwachszone größer ist als eine Oberflächenrauheit in der Einlasszone.

2. Einlasskanüle nach dem vorhergehenden Anspruch, **dadurch gekennzeichnet, dass** die erste Tangente einen Winkel zur Längsachse der Einlasskanüle zwischen 10° und 170°, vorzugsweise zwischen 30° und 160°, besonders bevorzugt zwischen 85° und 95°, insbesondere von 90°, oder zwischen 60° und 70° oder zwischen 110° und 130° oder zwischen 145° und 155° aufweist.

3. Einlasskanüle nach einem der vorhergehenden Ansprüche, **dadurch gekennzeichnet, dass** die Abrisskante durchgängig in Umfangsrichtung der Einlasskanüle verläuft.

4. Einlasskanüle nach einem der vorhergehenden Ansprüche, **dadurch gekennzeichnet, dass** die Einwachszone eine Texturierung aufweist, insbesondere dass die Einwachszone in einem Teilbereich oder vollständig eine Texturierung aufweist.

5. Einlasskanüle nach dem vorhergehenden Anspruch, **dadurch gekennzeichnet, dass** die Texturierung mittels eines Sinterverfahrens, eines 3D-Druckverfahrens, insbesondere zum Aufdrucken einer Titan-Texturierung, durch Aufschießen von Kügelchen, insbesondere von Titan-Kügelchen, und/oder durch Aufkleben eines Fließstoffes, insbesondere Velours, erfolgt.

6. Einlasskanüle nach einem der zwei vorhergehenden Ansprüche, **dadurch gekennzeichnet, dass** die Texturierung eine gemittelte Rautiefe von Rz > 16 µm aufweist.

7. Einlasskanüle nach einem der vorhergehenden Ansprüche, **dadurch gekennzeichnet, dass** die Einlasszone poliert ist, wobei die polierte Einlasszone insbesondere eine gemittelte Rautiefe von Rz < 1,5 µm aufweist.

8. Einlasskanüle nach einem der vorhergehenden Ansprüche, **dadurch gekennzeichnet, dass** entlang der Flussrichtung die Einwachszone konkav, konvex oder nicht gekrümmt ist, und die Einlasszone konvex oder nicht gekrümmt ist, wobei die Abrisskante einen Krümmungsübergang zwischen der Einwachszone und der Einlasszone bildet.

9. Einlasskanüle nach dem vorhergehenden Anspruch, **dadurch gekennzeichnet, dass** die Einwachszone mit einem Krümmungsradius von 2 bis 20 mm, bevorzugt 5, 10 oder 15 mm konkav oder konvex gekrümmt ist.

10. Einlasskanüle nach einem der zwei vorhergehenden Ansprüche, **dadurch gekennzeichnet, dass** die Einlasszone mit einem Krümmungsradius von 2 bis 20 mm, bevorzugt 5, 10 oder 15 mm konvex gekrümmt ist.

11. Einlasskanüle nach einem der drei vorhergehenden Ansprüche, **dadurch gekennzeichnet, dass** sich die erste Tangente an die Einlasszone und eine zweite Tangente an die Einwachszone an der Abrisskante, in einem Winkel zwischen 40° und 150°, insbesondere in einem Winkel von 90° schneiden.

12. Einlasskanüle nach einem der vorhergehenden Ansprüche, **dadurch gekennzeichnet, dass** sich beim Übergang von der Einwachszone zur Einlasszone ein Sprung in einer Wandschubspannung zwischen dem Fluid und der Oberfläche der Einlasskanüle von mindestens 100 % ergibt.

13. Einlasskanüle nach dem vorhergehenden Anspruch, **dadurch gekennzeichnet, dass** die Einlasskanüle eine Pumpenschnittstelle zur Verbindung der Einlasskanüle mit einer Fluidpumpe aufweist, und dass eine dritte Tangente senkrecht zur Abrisskante an die Einlasszone in einem der Pumpenschnittstelle benachbarten Bereich der Einlasszone einen Winkel zwischen 0° und 30° zur Längsachse der Einlasskanüle aufweist.

14. Einlasskanüle nach einem der vorhergehenden Ansprüche, **dadurch gekennzeichnet, dass** die Einlasskanüle eine Einlassöffnung in einem der Abrisskante benachbarten Bereich der Einlasszone aufweist, und dass in der Einlassöffnung ein Gitterelement angeordnet ist, welches die Einlassöffnung teilweise verschließt.

15. Fluidpumpensystem mit einer Einlasskanüle nach einem der vorhergehenden Ansprüche und einer Fluidpumpe, wobei die Pumpenschnittstelle der Einlasskanüle an den Pumpeneinlass der Fluidpumpe angeschlossen ist.
